(19) Europäisches Patentamt
European Patent Office
Office européen des brevets

(11) **EP 3 245 991 B1**

(12) **EUROPEAN PATENT SPECIFICATION**

(45) Date of publication and mention
of the grant of the patent:
**08.01.2020 Bulletin 2020/02**

(51) Int Cl.:
***A61F 13/49*** *(2006.01)* ***A61F 13/15*** *(2006.01)*
***A61F 13/511*** *(2006.01)* ***A61F 13/512*** *(2006.01)*

(21) Application number: **15894275.5**

(22) Date of filing: **13.08.2015**

(86) International application number:
**PCT/JP2015/072922**

(87) International publication number:
**WO 2016/194243 (08.12.2016 Gazette 2016/49)**

(54) **NON-WOVEN FABRIC FOR ABSORBENT ARTICLE**

VLIESSTOFF FÜR SAUGFÄHIGEN ARTIKEL

NON-TISSÉ POUR ARTICLE ABSORBANT

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **29.05.2015 JP 2015110963**

(43) Date of publication of application:
**22.11.2017 Bulletin 2017/47**

(73) Proprietor: **Unicharm Corporation
Ehime 799-0111 (JP)**

(72) Inventors:
• **UDA, Masashi
Kanonji-shi
Kagawa 769-1602 (JP)**
• **SAKAGUCHI, Satoru
Kanonji-shi
Kagawa 769-1602 (JP)**
• **MIYAMA, Takuya
Kanonji-shi
Kagawa 769-1602 (JP)**

(74) Representative: **Dolleymores
9 Rickmansworth Road
Watford, Hertfordshire WD18 0JU (GB)**

(56) References cited:
**EP-A1- 2 095 802        EP-A1- 2 277 485
EP-A1- 2 505 173        EP-A1- 2 612 961
WO-A1-2013/005782    WO-A1-2013/129167
JP-A- H0 458 951        JP-A- H05 317 358
JP-A- H08 302 555        JP-A- H09 299 402
JP-A- H10 211 232        JP-A- 2007 130 178
JP-A- 2011 120 661        JP-A- 2013 132 523**

**Description**

Technical Field

The present invention relates to a nonwoven fabric to be used in an absorbent article such as a disposable diaper, sanitary napkin or incontinence pad.

BACKGROUND ART

In an absorbent article such as a disposable diaper, for example, it is a major requirement that the nonwoven fabric used as a structural member, such as the top sheet, must be soft and have a good feel on the skin, since it is the section that comes into contact with the skin of the user.

In recent years, therefore, it has become common to employ nonwoven fabrics provided with multiple rows of raised sections and furrow sections provided in the spaces between adjacent raised sections, on the side that comes into contact with the skin. In such nonwoven fabrics, since relatively soft raised sections come into contact with the skin and the raised sections easily fit to the skin surface, the nonwoven fabric tends to easily feel soft.

Among such types of nonwoven fabrics, for the purpose of further improving feel on the skin by creating a delicate, soft tactile sensation, as described in PTL 1 for example, there exist ones having on the surfaces of arched raised sections, small isolated raised sections that are smaller than those raised sections. The nonwoven fabric described in PTL 1 reduces the contact area of the nonwoven fabric on the skin by the small raised sections, thereby tending to produce a dry feeling or a sliding feel when touching the nonwoven fabric, and improving the feel on the skin.

Citation List

Patent Literature

[PTL 1] Japanese Unexamined Patent Publication No. 2005-334374

EP2612961A1 discloses a non-woven fabric sheet having a front surface and an opposing surface thereto in the form of a back surface in the direction of thickness, and having ridges and grooves alternately formed so as to extend mutually in parallel in the longitudinal direction so as to form repeated undulations in the transverse direction. The non-woven fabric sheet has a bottom surface that contacts a horizontal surface when placed on the horizontal surface with the back surface. Sites where the height from the bottom surface is relatively high and low sites corresponding to the bottom surface are alternately formed along the longitudinal direction on the back surface of the grooves. Openings are formed at least in the sites where the height from the bottom surface is relatively high, and a space is provided below the openings.

WO 2013/005782 A1 relates to an absorbent article having a top sheet, a back sheet, an absorbent core and a cover sheet covering the absorbent core. The non-skin contact surface side of the top sheet has a plurality of back recesses (back surface concave portions) recessed toward the skin contact surface side. The non-skin contact surface side of the top sheet has an uneven shape by the periodic arrangement of the back recesses. The cover sheet enters the back recesses to provide a space between the cover sheet and the absorbent core.

SUMMARY OF INVENTION

TECHNICAL PROBLEM

However, while the nonwoven fabric described in PTL 1 has improved feel on the skin compared to a lack of small raised sections, due to the reduced contact area of the nonwoven fabric on the skin, when the skin slides against the surface of the nonwoven fabric, for example, the concavoconvex nature of the small raised sections can sometimes leave a rough tactile sensation as that of a dried nonwoven fabric surface, potentially making it impossible to achieve a delicate, soft, skin-friendly tactile sensation.

The technical problem of the invention is to provide a nonwoven fabric for an absorbent article that can produce a delicate, soft, skin-friendly tactile sensation when come into contact with the finger.

SOLUTION TO PROBLEM

In order to solve the above-mentioned problem, the nonwoven fabric for an absorbent article according to the present invention is as follows.

(1) A nonwoven fabric for an absorbent article, containing thermoplastic resin fibers, having a first surface and a second surface opposing the first surface, and comprising a plurality of raised sections protruding in a direction from the second surface to the first surface and a plurality of furrow sections depressed in the direction from the first surface to the second surface, wherein the raised sections are extended in a first direction of planar directions of the nonwoven fabric, while being provided at predetermined intervals in a second direction that is perpendicular to the first direction of the planar directions of the nonwoven fabric, the furrow sections are extended in the first direction, in the spaces between mutually adjacent raised sections in the second direction, and each includes a first hollow section comprising a first bottom section located further in the direction of the second surface than the location of the first surface at the top section of the raised section and a plurality of second hollow sections provided in a discontinuous manner in the first direction in the first hollow section, formed as depressions opening into the first bottom section and comprising second bottom sections located further in the direction of the second surface than the first bottom section, a distance between a top section of a raised section and a top section of another raised section adjacent to the raised section is 0.5 to 2 mm, and a mean fiber size of fibers forming at least the raised sections of the nonwoven fabric is 10 to 30 μm.

(2) The nonwoven fabric for an absorbent article according to the above (1), wherein an average interfiber distance of the fibers forming the raised sections is 50 to 150 μm.

This helps the fibers forming the raised sections to run along irregularities of fingerprints, allowing the fibers forming the raised sections to more stably infiltrate between the protrusions, to allow a more skin-friendly and moist tactile sensation to be obtained.

(3) The nonwoven fabric for an absorbent article according to the above (1) or (2), wherein the second hollow section has a perimeter wall section extended in a direction opposite to the direction in which the raised section protrude from the first bottom section, and the second bottom section disposed on an edge of the perimeter wall section in the direction opposite to the first bottom section so as to plug the edge.

This can more stably reduce the contact area of the first bottom section of the first hollow section with the skin, and impart a soft tactile sensation. In addition, since the second bottom section is at the location far from the raised section due to the perimeter wall section, when the skin comes into contact with the first surface, the second bottom section is less likely to come into contact with the skin, allowing the smooth feel of the first surface on the skin to be ensured.

(4) The nonwoven fabric for an absorbent article according to the above (3), wherein the perimeter wall sections of the second hollow section has a pair of first perimeter wall sections formed along the first direction, and a pair of second perimeter wall sections formed along the second direction, the first perimeter wall section has a hole section running through between the first surface and the second surface.

This allows the hole sections of the first perimeter wall sections of the perimeter wall sections to release the tension of the fibers forming the adjacent raised sections, thereby improving the freedom of movement of the raised sections as a whole or the fibers forming the raised sections. As a result, it is possible to further increase the flexibility of the raised sections and help the fibers of the raised sections follow irregularities in fingerprints, allowing a delicate, soft, skin-friendly tactile sensation to be more stably obtained.

(5) The nonwoven fabric for an absorbent article according to the above (4), wherein the hole section has a peripheral section formed without melting of the thermoplastic resin fibers, and the peripheral section includes broken ends of broken fibers that have broken ends formed by breakage among the thermoplastic resin fibers.

With this structure, even when human skin has come into contact with the peripheral sections of the hole sections, the absence of thermoplastic resin fibers hardened by melting in the peripheral sections suppresses any uncomfortable feeling due to stiffness or catching of the fibers and reduces any feeling of hardness or roughness from the nonwoven fabric.

(6) The nonwoven fabric for an absorbent article according to the above (4) or (5), wherein the hole section has some of the thermoplastic resin fibers passing through the interior space of the hole section.

With this structure, even if the skin comes into contact with the hole sections, the thermoplastic resin fibers in the interior spaces can reduce the borders or level differences between the hole sections and the first perimeter wall sections or second bottom sections of the second hollow sections. As a result, it is possible to maintain a smooth feel on the skin even if hole sections are present, thus making it possible to reduce any uncomfortable feeling on contact caused by the presence of the hole sections.

(7) The nonwoven fabric for an absorbent article according to any one of the above (4) to (6), wherein the hole section has an open area ratio in the interior space of 1 to 50%.

This can provide a suitable degree of freedom to the raised sections or the fibers of the raised sections to ensure flexibility, while also ensuring strength of the first perimeter wall sections in which the hole sections are provided, and can suppress any uncomfortable feeling from contacting the peripheral sections of the hole sections.

(8) The nonwoven fabric for an absorbent article according to any one of the above (4) to (7), wherein the hole sections are provided only at locations near the second bottom sections of the first perimeter wall sections.

This can separate the hole sections as far as possible from the raised sections and first bottom sections that are more likely to come into contact with the skin, therefore minimizing opportunity for the hole sections to be contacted by the skin and reducing any uncomfortable feeling or sensation of foreign object caused by the hole sections. As a result, it is possible to more stably ensure smoothness when the skin slides in the planar directions of the nonwoven fabric.

(9) The nonwoven fabric for an absorbent article according to any one of the above (1) to (8), wherein the second hollow section has a distance of 0.05 to 2 mm from the first surface of the first bottom section of the first hollow section to the first surface of the second bottom section.

This can ensure the fiber density during formation of the second bottom sections to minimize reduction in rigidity of the second bottom sections, while also ensuring rigidity of the perimeter wall sections of the second hollow sections and ensuring strength of the nonwoven fabric as a whole.

ADVANTAGEOUS EFFECTS OF THE INVENTION

According to the present invention, the mean fiber diameter of the fibers forming the raised sections is smaller than the widths between protrusions on fingerprints, and the distances between the top sections of adjacent raised sections are within a predetermined range, such that the fibers forming the raised sections easily run along the irregularities of fingerprints and the fibers forming the raised sections readily infiltrate between the protrusions. At the same time, the presence of the second hollow sections reduces the area of contact of the first bottom sections of the first hollow sections with the skin, so that a soft feel on the skin can be obtained. This allows a soft feel on the skin to be obtained for the nonwoven fabric as a whole, while also allowing the fibers forming the raised sections to stimulate the tactile organs on the flat sections between adjacent protrusions of fingerprints and on the side sections of the protrusions, to obtain a delicate, soft, skin-friendly and moist tactile sensation.

BRIEF DESCRIPTION OF THE DRAWINGS

Fig. 1 is a partial fracture perspective view schematically showing an embodiment of a nonwoven fabric for an absorbent article according to the invention.
Fig. 2 is a plan view schematically showing an embodiment of the nonwoven fabric for an absorbent article according to the invention.
Fig. 3 is a main part enlarged cross-sectional view along line I-I of Fig. 2.
Fig. 4 is (a) a schematic diagram of a fingerprint, and (b) a cross-sectional view for explaining the state that fibers forming raised sections of the nonwoven fabric for an absorbent article according to the invention come into contact with a fingerprint.
Fig. 5 is a diagram schematically showing an example of a production apparatus for production of the nonwoven fabric for an absorbent article according to the invention.
Fig. 6 is a main part enlarged perspective view schematically showing a pair of stretching rolls in a shaping apparatus.
Fig. 7 is a main part enlarged view schematically showing an arrangement of pins in a lower stretching roll.
Fig. 8 is a main part enlarged view showing the interlocked state of an upper stretching roll and lower stretching roll.

DESCRIPTION OF EMBODIMENTS

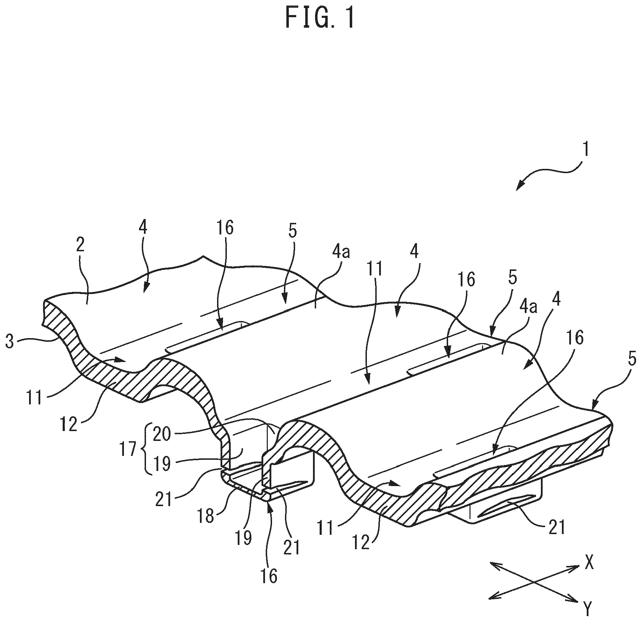

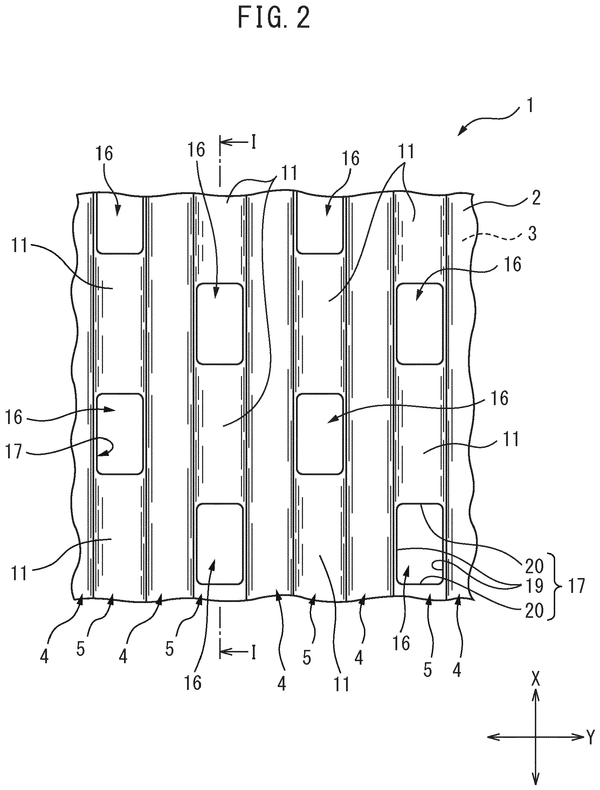

Fig. 1 to Fig. 3 show an embodiment of a nonwoven fabric for an absorbent article of the present invention, the nonwoven fabric 1 of this embodiment, having a first surface 2 and a second surface 3 opposing the first surface 2, including a plurality of raised sections 4 protruding toward the first surface 2 side (the upper side in Fig. 1 to Fig. 3) and a plurality of furrow sections 5 depressed toward the second surface 3 side, i.e. in the direction opposite to the direction in which the raised sections 4 protrude (the lower side in Fig. 1 to Fig. 3), and being used for an absorbent article that includes thermoplastic resin fibers. Also, the nonwoven fabric 1 has a first direction X in planar directions of the nonwoven fabric 1, and a second direction Y perpendicular to the first direction X in the planar directions of the nonwoven fabric 1.
The explanation in this embodiment will assume that the direction in which the raised sections 4 protrude is upward and the direction opposite to the direction in which the raised sections protrude is downward.
The nonwoven fabric of the present invention may be suitably used as a top sheet or anti-leakage wall of an absorbent article such as a disposable diaper, sanitary napkin, urine-absorbing pad, panty liner or the like, or in other words, as a sheet that is to be disposed on the side of the absorbent article that is to come into contact with the skin of the user. Alternatively, it may be used as a sheet to be attached onto the outer side of a back sheet of a disposable diaper or the like.

When the nonwoven fabric of the present invention is used in an absorbent article, the second surface of the nonwoven fabric is joined to each structural member of the absorbent article (for example, the absorbent body when used as a top sheet). In this case, it is highly preferred that the second surface side of the raised sections (especially the top sections) may not be joined.

Also according to the present invention, the thermoplastic resin composing the thermoplastic resin fibers in the nonwoven fabric may be a polyolefin, polyester, polyamide or the like. Examples of polyolefins include straight-chain low-density polyethylene (LLDPE), low-density polyethylene (LDPE), medium-density polyethylene (MDPE), high-density polyethylene (HDPE), polypropylene, polybutylene, and copolymers composed mainly of the foregoing (for example, ethylene-vinyl acetate copolymer (EVA), ethylene-ethyl acrylate copolymer (EEA), ethylene-acrylic acid copolymer (EAA) or an ionomer resin). Examples of polyesters include polyethylene terephthalate (PET), polytrimethylene terephthalate (PTT), polybutylene terephthalate (PBT), polylactic acid, polyesters of straight-chain or branched polyhydroxyalkanoic acids up to C20 such as and polyglycolic acid, copolymers composed mainly thereof, and copolymerized polyesters composed mainly of alkylene terephthalates copolymerized with a small amount of another component. Examples of polyamides include 6-nylon and 6,6-nylon. The fiber lengths will usually be 20 to 100 mm and are preferably 35 to 65 mm. The thermoplastic resin fibers may be subjected to hydrophilicizing treatment, the hydrophilicizing treatment being, for example, treatment utilizing a surfactant, hydrophilic agent or the like (for example, kneading of a surfactant into the fiber interiors, or coating of the fiber surfaces with a surfactant).

The nonwoven fabric of the present invention may further contain other constituent fibers in addition to thermoplastic resin fibers. Examples of other constituent fibers include natural fibers (for example, wool, cotton and the like), regenerated fibers (for example, rayon, acetate and the like), and inorganic fibers (for example, glass fibers, carbon fibers and the like). Also, the nonwoven fabric may be combined with composite fibers such as core/sheath fibers, side-by-side fibers and sea/island fibers, hollow type fibers; irregularly shaped fibers such as flat fibers, Y-shaped fibers or C-shaped fibers; solid crimped fibers such as latent crimped or developed crimped fibers, or split fibers that have been split by a physical load such as a water stream, heat, embossing or the like.

Furthermore, the nonwoven fabric 1 has an approximately wavy shape formed by alternately folding an unprocessed nonwoven fabric sheet, as the starting material, in the direction of the first surface 2 side and the direction of the second surface 3 side, i.e. upward and downward, and has a configuration such that the plurality of raised sections 4 are formed by the sections folded toward the first surface 2 side, i.e. upward, and the plurality of furrow sections 5 are formed by the sections folded in the direction opposite to the direction in which the raised sections 4 protrude, i.e. downward.

The raised sections 4 are extended continuously in the first direction X on in the planar direction (sheet plane) of the nonwoven fabric 1, while being arranged in a plurality of rows at a predetermined interval in the second direction Y. In this embodiment, each of the raised sections 4 is extended continuously in the first direction X, so as to be mutually substantially parallel with the other raised sections 4.

The furrow sections 5 are extended in the first direction X, in the spaces between adjacent raised sections 4, 4 in the second direction Y.

The furrow sections 5 have first hollow sections 11, each including: a first bottom section 12 located further in the direction of the second surface 3, i.e. downward, than the location of the first surface 2 at the top section 4a of the raised sections 4; and a plurality of second hollow sections 16 each provided in a discontinuous manner in the first direction X in each of the first hollow sections 11, and formed as a depression opening into the first bottom section 12.

The first hollow sections 11 are formed integrally with the raised sections 4 in the second direction Y. Also, a thickness at the largest section of the first bottom section 12 of the first hollow section 11, which is the maximum distance between the first surface 2 and second surface 3, are the greatest among the thickness of the nonwoven fabric 1, and thus the first bottom sections 12 are sections with excellent elasticity as a whole. The plurality of first hollow sections 11 formed in the nonwoven fabric 1 is all formed with mutually equal widths.

The second hollow sections 16 have approximately rectangular openings in planar view (as viewed from the top for this embodiment), and protrude overall to the lower side of the nonwoven fabric 1, including approximately cuboid interior spaces. Also, the second hollow sections 16 are disposed at a constant interval in the first direction X of each of the furrow sections 5, and more specifically in the first direction X of the first hollow sections 11, each of the second hollow sections 16 being formed in a mutually independent manner from the other second hollow sections 16.

Furthermore, the second hollow sections 16 include: wall-like perimeter wall sections 17 oriented downward, which is the direction opposite to the direction in which the raised sections 4 protrude from the first bottom sections 12; and second bottom sections 18 located further in the direction of the second surface 3, i.e. downward, than the first bottom sections 12, and disposed on the edges of the perimeter wall sections 17 opposite to the first bottom sections 12, i.e. the edges on the lower side, so as to plug those edges.

The second bottom sections 18 are formed by compression of the fibers forming the nonwoven fabric 1 in the up-down direction, and have the highest fiber density within the nonwoven fabric 1, and also the greatest rigidity.

In addition, the first surfaces 2 of the second bottom sections 18 (i.e. the inner side surfaces of the second hollow sections 16) and the second surfaces 3 are formed in an approximately planar manner overall.

According to the present invention, the second hollow sections are provided in the nonwoven fabric in order to reduce to a minimum the likelihood that the bottoms of the furrow sections, and more specifically the first bottom sections of the first hollow sections, will come into contact with the skin, and in order to minimize the contact area even when the first bottom sections have come into contact with the skin.

In other words, when the nonwoven fabric of the present invention is used as a top sheet in an absorbent article, for example, the second surface of the nonwoven fabric is joined to the absorbent body of the absorbent article, and in that state, the raised sections most readily come into contact with the skin, followed in order by the first bottom sections of the first hollow sections. Since the raised sections (especially the top sections) are essentially unjoined with the absorbent body and have increased flexibility, the raised sections are preferably given greater opportunity to come into contact with the skin than the first bottom sections. On the other hand, as a nonwoven fabric to be used in a top sheet or the like, the nonwoven fabric as a whole will feel more flexible if it has a lower contact area that comes into contact with the skin.

According to the present invention, therefore, the second hollow sections are provided to form sections where the first bottom sections are not present, thereby further reducing the sections that contact with the skin at the first bottom sections of the first hollow sections, and reducing to a minimum the likelihood that the skin will come into contact with the first bottom sections to facilitate their contact with the raised sections, while also greatly minimizing the contact area even if the skin comes into contact with the first bottom sections.

In addition, the second hollow sections are provided on the first bottom sections because providing the second hollow sections in the first bottom sections of the first hollow sections that are less likely to come into contact with the skin than the raised sections, ensures distance between the raised sections and the second bottom sections and reduces to a minimum any opportunity for the second hollow sections to come into contact with the skin. This can minimize any uncomfortable feeling or sensation of foreign object caused by contact with the rigid second hollow sections that have higher fiber density and higher rigidity than the other sections.

The second hollow section of the present invention has a dimension from the height of the first bottom section of the first hollow section (the location nearest the second bottom section, in this case) on the first surface (the top surface in this case) to the height of the second bottom section of the second hollow section on the first surface of preferably 0.05 to 2 mm, more preferably 0.075 to 1.5 mm and even more preferably 0.1 to 1 mm, although this will depend on the thickness of the nonwoven fabric.

If the dimension of the height of the first bottom section of the first hollow section on the first surface to the height of the second bottom section of the second hollow section on the first surface is less than 0.05 mm, the fiber density will be low and it will be difficult to ensure rigidity during formation of the second bottom sections, and the strength of the nonwoven fabric in the thickness direction may be insufficient. If it is greater than 2 mm, conversely, when the second bottom sections are attached to other members of the absorbent article, such as an absorbent body, or another nonwoven fabric, film or the like, the perimeter wall sections of the second hollow sections will extend too far lowering the strength in the thickness direction of the nonwoven fabric, while also potentially resulting in a hard feel when compressed.

Furthermore, as regards the relationship between the second hollow section and the raised section, the dimension from the height of the first bottom section of the first hollow section on the first surface to the height of the second bottom section of the second hollow section on the first surface is preferably 10 to 80%, more preferably 15 to 70% and even more preferably 20 to 60% of the dimension from the height of the first bottom section of the first hollow section (the location nearest the second bottom section in this case) on the first surface and the height of the top section of the raised section on the first surface side.

If the dimension from the height of the first bottom section of the first hollow section on the first surface side to the height of the second bottom section of the second hollow section on the first surface side is less than 10% of the dimension from the height of the first bottom section of the first hollow section on the first surface side to the height of the top section of the raised section on the first surface side, then it will not be possible to sufficiently ensure space for formation of the hole sections on the perimeter wall sections, resulting in inadequate formation of the hole sections and inability to obtain flexibility for the raised sections and therefore for the nonwoven fabric. If it is 80%, conversely, the second hollow sections will be too deep and the strength of the perimeter wall sections of the hollow sections will be reduced, tending to result in napping and potentially resulting in a poorer feel on the skin.

Also, the lengths of the second hollow section in the first direction and second direction will depend on the width of the furrow section, i.e. the distance between adjacent raised sections, but are preferably 0.25 to 5 mm, more preferably 0.5 to 3 mm and even more preferably 0.75 to 2 mm.

If the lengths of the second hollow section in the first direction and second direction are less than 0.25 mm, the second hollow section may likewise be too small, and formation of the second bottom section in particular may be inadequate, potentially causing the second hollow section to essentially cease to function. If they are greater than 5 mm, conversely, the second hollow section will be too large, potentially creating an uncomfortable feeling or sensation of foreign object on the skin by the hollow sections.

Also, the perimeter wall section 17 has a pair of first perimeter wall sections 19, 19 formed along the first direction X and a pair of second perimeter wall sections 20, 20 formed along the second direction Y. The pair of first perimeter wall sections 19, 19 is disposed at mutually facing locations, while the pair of second perimeter wall sections 20, 20 is also disposed at mutually facing locations.

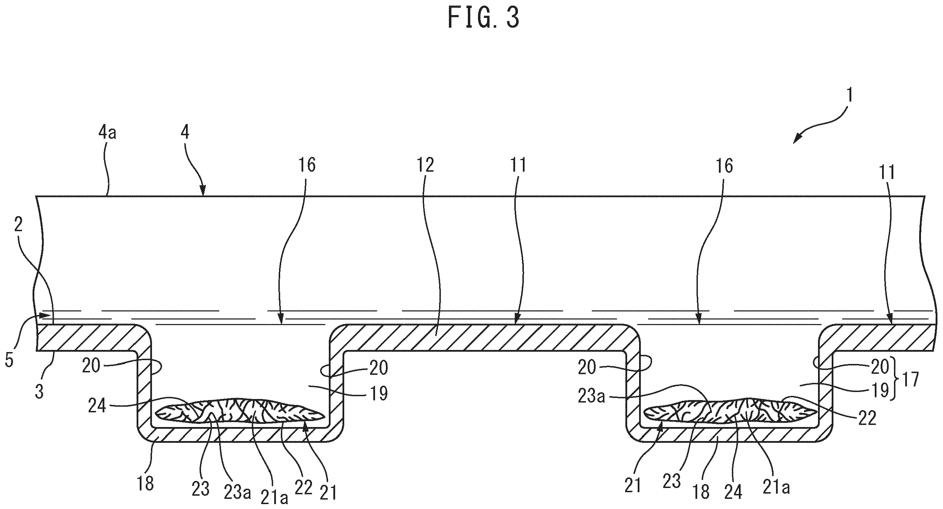

As shown in Fig. 1 and Fig. 3, the pair of first perimeter wall sections 19, 19 has hole sections 21 formed therein running from the interior space of the first hollow section 11 to the second surface 3.

According to this embodiment, one hole section 21 is provided for each of the pair of first perimeter wall sections 19, 19, and the hole sections 21 are formed at locations near the second bottom section 18 of the first perimeter wall sections 19 (and therefore two hole sections 21 are present for each second hollow section 16). On the other hand, the pair of second perimeter wall sections 20, 20 does not have things corresponding to the hole sections 21, each of the second perimeter wall sections 20 being directly connected to the second bottom section 18.

In this embodiment, the hole sections 21 are provided in the first perimeter wall sections 19 in order to release tension of the fibers of the raised sections 4 adjacent to the furrow sections 5 in which the hole sections 21 are provided. This can increase the freedom of movement of the raised sections 4 as a whole or of the fibers forming the raised sections 4, and can improve the flexibility of the raised sections 4, and more specifically the flexibility in the thickness direction of the raised sections 4 (the direction from the first surface 2 to the second surface 3), and improve the flexibility when the skin slides in the first direction X or second direction Y (particularly the second direction Y) of the nonwoven fabric 1, helping to ensure a smooth tactile sensation. As a result, the raised sections 4 are provided with both an excellent hard/soft feeling (excellent softness in the thickness direction) and an excellent rough/smooth feeling in the first direction X and second direction Y (excellent smoothness on the surface of the nonwoven fabric 1 (particularly in the second direction Y)), to allow an excellent hard/soft feeling and rough/smooth feeling to be ensured for the nonwoven fabric 1 as a whole, thereby allowing a soft feel on the skin to be achieved.

Conversely, hole sections 21 are not provided on the second perimeter wall sections 20 in order to reduce the level difference due to the presence of the second hollow sections 16 from catching on the skin when the skin slides in the first direction X of the nonwoven fabric 1, i.e. in the direction in which the raised sections 4 or furrow sections 5 are extended, to ensure smoothness in the first direction X of the nonwoven fabric 1. That is, since the second perimeter wall sections 20 are continuous with the first bottom sections 12 and second bottom sections 18 and integral without seams, the skin does not significantly sense the level difference of the first bottom sections 12 due to the presence of the second hollow sections 16 and smoothly and easily moves along the raised sections 4 and furrow sections 5, when the skin slides in the first direction X of the nonwoven fabric 1. This can ensure smoothness in the first direction X of the nonwoven fabric 1 due to the flexibility of the raised sections 4 or flexibility of the fibers.

In addition, the hole sections 21 are provided at locations of the first perimeter wall sections 19 near the second bottom sections 18 in order for the hole sections 21 to be as far as possible from the raised sections 4 or first bottom sections 12 that tend to come into contact with the skin, thereby minimizing opportunity for the hole sections 21 to come into contact with the skin and reducing any uncomfortable feeling or sensation of foreign object. This can more stably ensure smoothness when the skin slides in the planar direction of the nonwoven fabric 1.

In addition, as shown in Fig. 3, the hole section 21 includes a peripheral section 22 formed by breakage of the thermoplastic resin fibers in the nonwoven fabric 1, without melting the thermoplastic resin fibers. The peripheral section 22 of the hole section 21 includes, among the thermoplastic resin fibers, the broken ends 23a of broken fibers 23 having broken ends 23a formed by breakage of the thermoplastic resin fibers, and does not include thermoplastic resin fibers that have been hardened by melting or the like. The broken ends 23a of the broken fibers 23 are some of the thermoplastic resin fibers in the first perimeter wall section 19, and they are formed by breakage by pulling or physical cutting of the thermoplastic resin fibers in the first direction X. Thus, instead of melted and rounded fiber ends, increased fiber diameters and hardened fibers as when thermoplastic resin fibers have melted, the broken fibers 23 which have been torn are tapered, or else have virtually no change in fiber diameter.

As a result, even when human skin has been in contact with the peripheral sections 22 of the hole sections 21, the absence of thermoplastic resin fibers hardened by melting in the peripheral sections 22 suppresses any uncomfortable feeling due to stiffness or catching of the fibers and reduces any feeling of hardness or roughness from the nonwoven fabric 1.

In addition, the interior space 21a of the hole section 21 has a combination of thermoplastic resin fibers 24 passing through the interior space 21a and some broken fibers 23 with the broken ends 23a extending into the interior space 21a, so that the space is not completely open. Thus, since the hole section 21 has some of the thermoplastic resin fibers passing through or extending into the interior space 21a, even if the skin comes into contact with the hole sections 21, the thermoplastic resin fibers in the interior space 21a can reduce the borders or level differences between the first perimeter wall sections 19 of the second hollow sections 16 or the second bottom sections 18 and the hole sections 21. This makes it is possible to maintain a smooth feel on the skin even though the hole sections 21 are present, thus helping to reduce any differences in tactile sensation caused by the presence of the hole sections 21 and reducing any uncomfortable feeling on contact.

Incidentally, the open area ratio of the interior space of the hole section is preferably 1 to 50%, more preferably 1.5 to 35% and even more preferably 2.5 to 20%.

If the open area ratio of the interior space of the hole section is less than 1%, the open area ratio will be too low, making it impossible to impart freedom to the raised sections or the fibers of the raised sections, or to adequately ensure flexibility for the raised sections. If it is 50% or greater, conversely, the strength of the first perimeter wall sections in which the hole sections are formed will tend to be reduced, and the borders of the peripheral sections of the hole sections may potentially be felt. However, the open area ratio of the interior space of the hole section may be outside of this range and set as desired, depending on the type of absorbent article and its purpose of use, etc.

Also as regards the dimension of the hole section, the length of the largest section of the hole section (the length in the first direction, in the case of this embodiment) will depend on the dimension of the second hollow section but are preferably 0.25 to 5 mm, more preferably 0.5 to 3 mm and even more preferably 0.75 to 2 mm.

If the length of the largest section of the hole section is less than 0.25 mm, formation of the hole sections will be inadequate and it will not be possible to ensure flexibility of the second hollow sections, while it will also be impossible to ensure adequate flexibility of the raised sections without lowering the tensile force of the fibers of the raised sections. If it is greater than 5 mm, conversely, the hole sections will be too large and the peripheral sections of the hole sections will be prone to napping, thereby tending to produce an uncomfortable feeling or sensation of foreign object by the hole sections, and risking a poorer feel on the skin by the nonwoven fabric.

The maximum length in the height direction of the hole section will depend on the depth of the second hollow section, but it is preferably 0.1 to 5 mm, more preferably 0.25 to 3 mm and even more preferably 0.5 to 2 mm.

If the maximum length in the height direction of the hole section is less than 0.1 mm, formation of the hole section will be inadequate and in some cases it will not be possible to ensure flexibility of the second hollow section, while it will also be impossible to ensure adequate flexibility of the raised section because the tensile force of the fibers of the raised section is not lowered. If it is greater than 5 mm, conversely, the peripheral section of the hole section will likewise be too large and the peripheral sections will be prone to napping, thereby tending to produce an uncomfortable feeling or sensation of foreign object by the hole section, and potentially impairing the feel on the skin by the nonwoven fabric.

Incidentally, the fibers 4b forming at least the raised sections 4 in the nonwoven fabric 1 have a mean fiber size of 10 to 30 $\mu$m.

According to the present invention, the mean fiber size of the fibers forming the raised sections is 10 to 30 $\mu$m in order to allow a delicate, soft, skin-friendly and moist tactile sensation to be obtained when skin of the finger comes into contact with the raised sections. The mean fiber size of the fibers forming the raised sections is a very important element for obtaining a nonwoven fabric with a delicate, soft, skin-friendly and moist tactile sensation.

That is, when touching the surface of an object with the finger, a human perceives whether the surface of the object is smooth or rough by the relationship between the distance (the pitch) between adjacent convexities among the irregularities on the surface of the object and the distance between the adjacent protrusions of the fingerprint. In other words, when the convexities on the surface of the object infiltrate between the protrusions of the fingerprint, stimulating the flat sections which are depressions between the protrusions of the fingerprint or the side sections other than the tips of the protrusions, tactile organs on the finger are stimulated and a delicate, soft, smooth tactile sensation is obtained.

Generally, human fingerprints are said to have a distance between adjacent protrusions of about 460 $\pm$15 $\mu$m. According to the present invention, therefore, the raised sections that are most likely to come into contact with skin in the nonwoven fabric are formed by fibers with a mean fiber size that is sufficiently smaller than the distance between adjacent protrusions of fingerprints, so that the fibers forming the raised sections easily infiltrate between the protrusions of fingerprints when a finger has touched the raised sections of the nonwoven fabric.

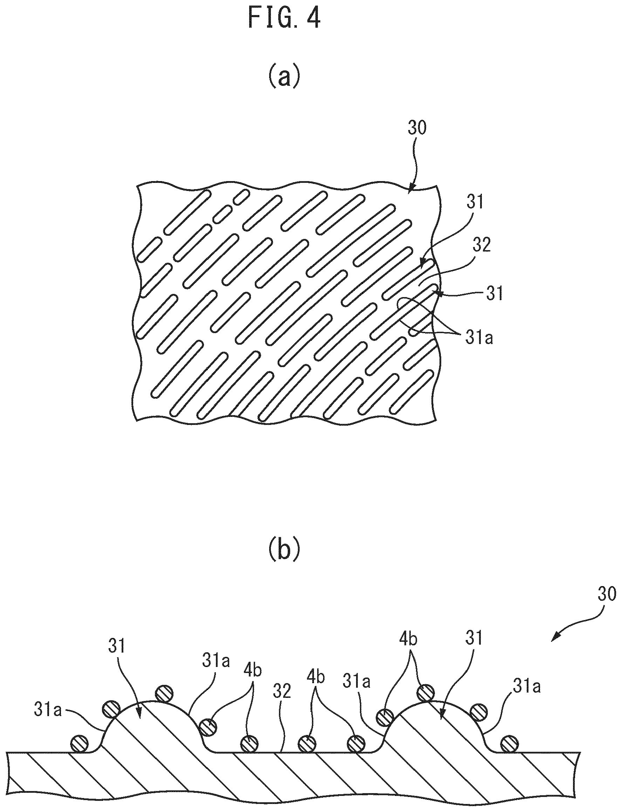

As a more detailed explanation, as shown in Fig. 4(a), the human finger 30 has a fingerprint formed by a plurality of protrusions 31 and depressed flat sections 32 between the protrusions 31. When the raised sections 4 comes into contact with the finger 30, since the mean fiber size of the fibers 4b forming the raised sections 4 is smaller than the distance between adjacent protrusions 31 of the finger 30, the fibers 4b forming the raised sections 4 infiltrate between the adjacent protrusions 31, 31 of the fingerprint of the finger 30, as shown in Fig. 4(b).

As a result, a plurality of the fibers 4b forming the raised sections 4 comes into contact with the flat sections 32 or the side sections 31a of the protrusions 31 on the finger 30, stimulating tactile organs on the finger 30 and thus producing a delicate, soft, skin-friendly and moist tactile sensation.

If the mean fiber size of the fibers forming the raised sections is less than 10 $\mu$m, the bulk of the nonwoven fabric will be deficient, while the fiber density will tend to increase, potentially impairing the flexibility. The strength of the nonwoven fabric as a whole will also be reduced. If it is greater than 30 $\mu$m, conversely, it may be difficult to come into contact with the regions between adjacent protrusions, and particularly the flat sections, of the fingerprint, potentially making it difficult to obtain a delicate sensation and impossible to obtain a moist tactile sensation.

The mean fiber size of the fibers forming the raised sections is more preferably 10 to 20 $\mu$m.

Measurement of the mean fiber size of the fibers forming the raised sections can be accomplished by obtaining an enlarged image of the fibers forming the raised sections using an electron microscope or other microscope (for example, VHX-2000 by Keyence Corp.), and measuring the distance between 2 points corresponding to the diameter of fibers in the images. The number of measured fibers is 100 as a general rule, and the average is defined as the mean fiber size.

The nonwoven fabric 1 also has an average interfiber distance of 50 to 150 $\mu$m between the fibers forming the raised sections 4.

According to the present invention, the average interfiber distance of the fibers forming the raised sections is 50 to 150 $\mu$m in order to further help the locations of the fibers forming the raised sections to conform to the distance between adjacent protrusions of the fingerprint, and cause the fibers forming the raised sections to maximally come into contact along the irregularities of the fingerprint. This allows the fibers to more stably and reliably infiltrate between the protrusions of the fingerprint, and therefore a skin-friendly, moist tactile sensation is more reliably and stably obtained when the raised sections are touched with the finger.

If the average interfiber distance between the fibers forming the raised sections is less than 50 $\mu$m, the interfiber distances will be too close, resulting in difficult movement of the fibers, and making it difficult for the fibers forming the raised sections to run along the irregularities of the fingerprint. If it is greater than 150 $\mu$m, conversely, the fibers forming the raised sections will not easily infiltrate into the adjacent protrusions of the fingerprint and the number of infiltrating fibers will be reduced, such that the delicate sensation may not be obtained and skin-friendly spacing may be lost.

The average interfiber distance of the fibers forming the raised sections is more preferably 60 to 130 $\mu$m and even more preferably 70 to 110 $\mu$m.

The average interfiber distance of the fibers forming the raised sections can be measured, for example, using a mercury porosimeter (product of Shimadzu Corp.), based on the mercury porosimetry method (JIS R 1655). The mercury porosimetry method provides information relating to the structure of a nonwoven fabric, by measuring the pressure applied to mercury injected among constituent fibers of the nonwoven fabric that is to be measured with mercury, and the volume of mercury pressed into the spaces among the constituent fibers of the nonwoven fabric.

Specifically, the interfiber distance of a nonwoven fabric can be measured by the following procedure using a mercury porosimeter.

1) The nonwoven fabric to be measured is cut to a size of 24 mm $\times$ 15 mm to prepare a measuring sample. Three measuring samples are prepared.

2) The three measuring samples are set in the sample cell of the mercury porosimeter (product of Shimadzu Corp.) without overlapping, and then the pressure on the mercury is gradually varied while measuring the volume (i.e., the pore volume) of mercury that has been pressed into the spaces (i.e. the pores) among the constituent fibers of the nonwoven fabric. The measurement is conducted in an environment of 22°C, 65% RH.

3) The diameter D ($\mu$m) of the pores (that is, the interfiber distance ($\mu$m) of the nonwoven fabric) is calculated by the following formula (1), and the relationship between the diameter D and the pore volume is obtained as a distribution curve for the diameter D (differential and integral curves). The measuring conditions for formula (1) below are a mercury surface tension of 0.483 N/m, a contact angle of 130°, and a mercury pressure of 0 to 414 MPa (absolute pressure).

[Formula 1]

$$D = -4\gamma \cos\theta / P \qquad \cdot \cdot \cdot (1)$$

(In the formula, D represents the diameter (interfiber distance), $\gamma$ represents the surface tension of mercury, 0 represents the contact angle and P represents the pressure.)

4) Based on the obtained distribution curve for the diameter D, the ratio of the volume of pores with diameter D of 50 to 150 $\mu$m with respect to the total volume of pores of diameter D of 0 to 500 $\mu$m is calculated.

5) The procedure of the above (2) to (4) is repeated 3 times, and the average value for the obtained volume ratios is defined as the ratio (%) of interfiber distance of 50 to 150 $\mu$m among the total.

The following method may also be used, as another method for measuring the average interfiber distance of the fibers forming the raised sections.

Specifically, the nonwoven fabric to be measured is cut to a 5 cm-square shape as a sample, and the 3D image compositing function of a microscope (VHX-2000 by Keyence Corp., lens: VH-Z20W open aperture) is used to obtain a 200x enlarged image with the focus set from the surface of the sample to a depth of 100 $\mu$m, the outer sides of the fibers matching the focus based on the enlarged image are extracted, and the surfaces formed therein are considered to be the interfiber space. The area-measuring function of the microscope is used to determine the area occupied by the interfiber space. An arbitrary location on the sample is selected and the area of the interfiber space in the selected region is measured at 100 locations. The interfiber space diameter (r) is determined by the following formula (2) based on the area A [interfiber space area (mean value)] per interfiber space 1 in the selected region, and the interfiber space diameter (r) is defined as the interfiber distance.

[Formula 2]

$$(r) = 2 \cdot \sqrt{A / \pi} \qquad (2)$$

Also, the nonwoven fabric 1 has a spacing between adjacent raised sections 4, 4 and more specifically, a distance between the top sections 4a of any raised section 4 and the top sections 4a of the other raised sections 4 adjacent to that raised section 4 (the pitch of the raised sections), of 0.5 to 2 mm.

According to the invention, the distance between the top section of a raised section and the top section of the other raised section adjacent to that raised section is 0.5 to 2 mm, so that when a finger has touched the nonwoven fabric, virtually all of the protrusions of the fingerprint that has infiltrated between the raised sections reliably come into contact with the nonwoven fabric between the raised sections, thereby facilitating stimulation of the side sections of the protrusions. In addition to the above-mentioned mean fiber size of the fibers forming the raised sections, the distance between the top section of the raised section and the top section of the other raised section adjacent to that raised section is also a very important element for obtaining a nonwoven fabric with a delicate, soft, skin-friendly and moist tactile sensation.

In other words, whether or not a person touching a nonwoven fabric with the finger perceives a delicate, soft, skin-friendly and moist tactile sensation for the nonwoven fabric depends on whether or not the side sections of the protrusions of the fingerprint are stimulated. In addition, stimulation of the side sections of the protrusions can occur not only when the fibers forming the raised sections have infiltrated between the protrusions of the fingerprint, naturally, but also when the side sections of the protrusions of the fingerprint have directly come into contact with the surface of the nonwoven fabric when the finger touches the nonwoven fabric.

When a finger has touched the nonwoven fabric, the finger usually infiltrates not only the raised sections but also between the raised sections, and when this occurs, if the protrusions of the fingerprint can also enter between the raised sections, and virtually all of the protrusions (especially the side sections) can be stimulated, then a delicate, soft, skin-friendly and moist tactile sensation will be stably obtained for the nonwoven fabric.

As mentioned above, the human fingerprint has a distance of about $460 \pm 15$ $\mu$m between adjacent protrusions, and therefore if the distance between the top section of the raised section and the top section of other raised section adjacent to the raised section is less than 0.5 mm, the protrusions of the fingerprint will not easily infiltrate between the raised sections, or in some cases will not infiltrate at all. Furthermore, if the distance between the top section of the raised section and the top section of other raised section adjacent to the raised section is greater than 2 mm, the protrusions of the fingerprint may infiltrate between the raised sections but depending on the location, such as at the deepest location of the furrow section, for example, it may be difficult for the protrusions of the fingerprint to come into contact with the nonwoven fabric. In this case, since it is possible that stimulation of the protrusions of the fingerprint and especially the side sections of the protrusions may be weak or nonexistent, it may not be possible to adequately stimulate virtually all of the protrusions that have infiltrated between the raised sections, and it may not be possible to obtain a delicate, soft, skin-friendly and moist tactile sensation for the nonwoven fabric.

According to the present invention, therefore, the distance between the top section of the raised section and the top section of other raised section adjacent to the raised section is 0.5 to 2 mm.

Furthermore, if the distance between the top section of the raised section and the top section of other raised section adjacent to the raised section is less than 0.5 mm, the distance between the top section of the raised section and the top section of its adjacent raised section will be too short, so that the nonwoven fabric cannot be considered to have formed a concavoconvex structure, and as a result, the contact area with the skin by the raised sections may not be significantly reduced, and the feel on the skin can potentially be impaired.

Conversely, if the distance between the top section of the raised section and the top section of other raised section adjacent to the raised section is greater than 2 mm, the distance between the top section of the raised section and the top section of its adjacent raised section will be too great, increasing the possibility that the finger will come into contact with the first hollow sections when the first surface of the nonwoven fabric has been stroked with a finger, and as a result, the likelihood of coming into contact with the second bottom sections of the second hollow sections, which have high fiber density and high rigidity, will increase, impairing the flexible tactile sensation and resulting in a less skin-friendly feel.

The distance between the top section of the raised section and the top section of other raised section adjacent to the raised section can be measured by the following method, for example.

A sharp blade is prepared (for example, a standard replacement blade HA-100NB for a HA-7NB cutter knife (trade name) by Kokuyo Co., Ltd.) and used to cut the nonwoven fabric.

After standing for 24 hours after cutting, a sheet cross-section is enlarged 5x with a microscope by Keyence Corp., and the distance between the top sections of adjacent raised sections is measured along the Y-axial direction. During this time, if the top section of the raised section has a constant width in the Y-axial direction, the center of the constant width in the Y-axial direction is considered to be the top section for measurement.

Also, the distance between the top sections of adjacent raised sections along the Y-axial direction is measured at 10 locations, and the mean value is defined as the distance between the top section of the raised section and the top section of other raised section adjacent to the raised section.

A method for producing the nonwoven fabric for an absorbent article 1 having the above-mentioned configuration will be described below.

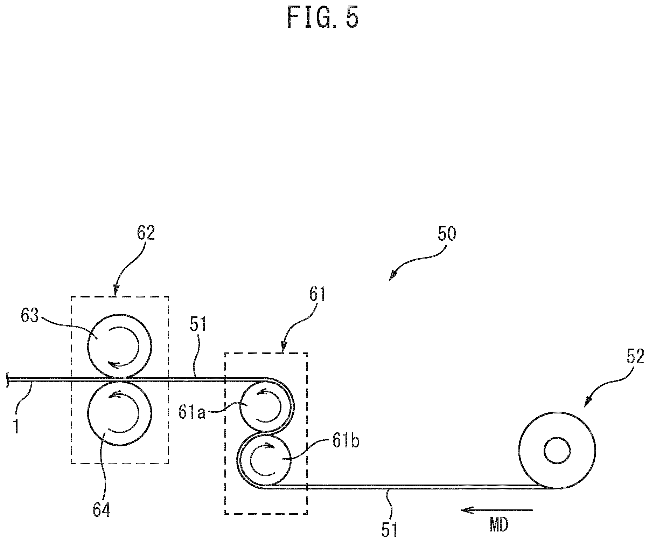

Fig. 5 to Fig. 8 show an example of a production apparatus for production of the nonwoven fabric 1, the production apparatus 50 including: an unwinding device 52 that has the nonwoven fabric 51 to be processed for producing the nonwoven fabric 1, the nonwoven fabric 51 having been wound into a roll, and that unwinds the nonwoven fabric 51 to be processed in the machine direction MD; and a preheater 61 that preheats the nonwoven fabric 51 to be processed, the nonwoven fabric 51 having been formed by the unwinding device 52. In addition, it also includes a shaping apparatus 62 that performs shaping to stretch the preheated nonwoven fabric 51 to be processed and form the raised sections 4 and furrow sections 5 (including the first hollow sections 11 and second hollow sections 16).

Examples for the method of producing the nonwoven fabric to be processed to be unwound by the unwinding device include a method of forming a web (fleece) and physically or chemically bonding the fibers together, where the method of forming the web includes, for example, a spunbond method, a dry method (a carding method, a meltblown method and an airlaid method) and a wet method, and the method of bonding includes, for example, a thermal bond method, a chemical bond method, a needle punching method, a stitch bond method and a spunlace method.

Also, from the viewpoint of the concealing property for excreted fluids when used as an absorbent article, the fibers used in the nonwoven fabric to be processed may be opaque, and especially highly whitening fibers, and for example, a light transmittance inhibitor that produces opaqueness may be used. Inorganic fillers are examples of the light transmittance inhibitors. Examples of such inorganic fillers include titanium oxide, calcium carbonate, talc, clay, kaolin, silica, diatomaceous earth, magnesium carbonate, barium carbonate, magnesium sulfate, barium sulfate, calcium sulfate, aluminum hydroxide, magnesium hydroxide, zinc oxide, calcium oxide, alumina, mica, glass powder, white sand balloons, zeolite and white silicate clay. These may also be included in combinations of two or more. Titanium dioxide is particularly preferred from the viewpoint of general process utility during fiber production steps.

Also, the thermoplastic resin fibers may have additives such as antioxidants, light stabilizers, ultraviolet absorbers, neutralizers, nucleating agents, epoxy stabilizers, lubricants, antimicrobial agents, flame retardants, antistatic agents, pigments or plasticizers, added as necessary. The thermoplastic resin fibers are preferably subjected to hydrophilicizing treatment with a surfactant, hydrophilic agent or the like.

The basis weight of the nonwoven fabric to be processed will usually be 10 to 100 $g/m^2$, and is preferably 15 to 75 $g/m^2$ and more preferably 20 to 50 $g/m^2$. The thickness of the nonwoven fabric to be processed will usually be 0.1 to 5 mm and is preferably 0.5 to 3 mm and more preferably 0.8 to 2 mm.

In this embodiment, the preheater 61 includes a pair of upper and lower heating rolls 61a, 61b, and wraps the nonwoven fabric 51 to be processed that has been conveyed around the rotating lower heating roll 61b, heats it at one side, and after that passes it to the rotating upper heating roll 61a, allowing another side of the nonwoven fabric 51 being processed that has not been heated by the lower heating roll, to be heated by the heating roll 61a.

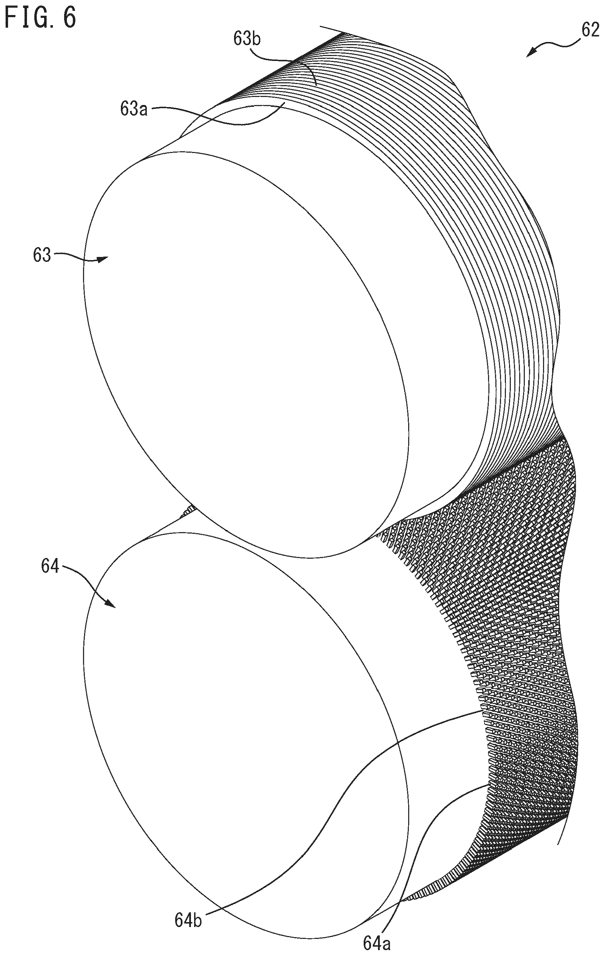

The shaping apparatus 62 also includes a pair of upper and lower stretching rolls 63, 64, and as shown in Fig. 6, the upper stretching roll 63 includes ridges 63a arranged in a plurality of rows that are mutually parallel around the outer peripheral surface of the upper stretching roll 63, disposed at a constant interval in the widthwise direction of the roll, and a plurality of rows of grooves 63b provided between adjacent ridges 63a, 63a.

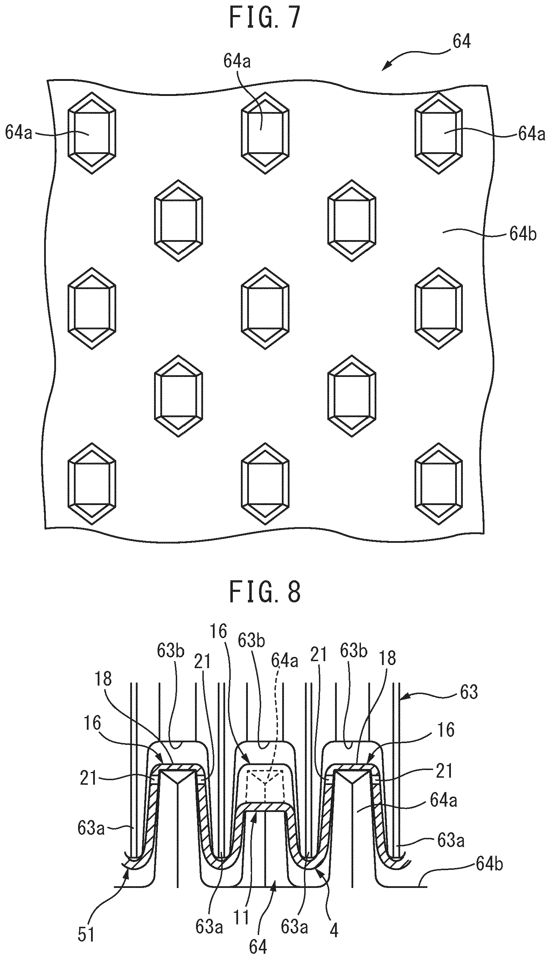

Also, the lower stretching roll 64 includes, around the outer peripheral surface, a plurality of pins 64a provided so as to interlock with the grooves 63b of the upper stretching roll 63, and hollow sections 64b that interlock with the ridges 63a. As shown in Fig. 6, the pins 64a are disposed at a constant interval in the widthwise direction of the roll so as not to come into contact with the ridges 63a of the upper stretching roll 63, while being linearly disposed at an approximately constant interval along the outer peripheral surface, with respect to the circumferential direction of the roll. Also, as shown in Fig. 7, the lower stretching roll 64 of this embodiment has a configuration in which a plurality of pins 64a are disposed in a zigzag fashion around the outer peripheral surface of the lower stretching roll 64.

When the production apparatus 50 having this configuration is used in a method for producing the nonwoven fabric for an absorbent article 1, a preheating step in which preheating is applied to the nonwoven fabric 51 to be processed that has been unwound from the unwinding device 52, and a shaping step in which the nonwoven fabric 51 to be processed that has passed through the preheating step is stretched and shaped, are carried out in that order.

The preheating step makes the nonwoven fabric 51 to be processed that has been unwound from the unwinding device 52 and conveyed along the machine direction MD come into contact with the outer peripheral surfaces of the rotating pair of upper and lower heating rolls 61a, 61b of the preheater 61, in that order, to heat both sides of the sheet surfaces of the nonwoven fabric 51 to be processed for preheating.

The preheating temperature will depend on the type of thermoplastic resin fibers composing the nonwoven fabric to be processed, and for example, heating is preferably performed at or above the temperature of initial softening of the thermoplastic resin fibers used in the nonwoven fabric to be processed, and a temperature below the melting point.

If the preheating temperature is at or above the melting point, the thermoplastic resin fibers will melt and harden, thus impairing the soft feel on the skin. Also if it is below the temperature of initial softening of the thermoplastic resin fibers, joining between the thermoplastic resin fibers will be maintained, making it difficult to shape the nonwoven fabric to be processed in the subsequent shaping step, and making it difficult to form suitable irregularities. For example, in the case of core-sheath composite fibers of polyethylene terephthalate (PET) and high-density polyethylene (HDPE), for example, the temperature of the outer peripheral surfaces of the heating rolls is preferably about 60 to 120°C.

The shaping step inserts the nonwoven fabric 51 to be processed that has passed through the preheating step and been conveyed between the engaged and rotating pair of upper and lower stretching rolls 63, 64 of the shaping apparatus 62, to stretch and shape the nonwoven fabric 51 to be processed between the ridges 63a and grooves 63b of the engaged upper stretching roll 63 and the hollow sections 64b and pins 64a of the lower stretching roll 64. To facilitate shaping when carrying out the shaping step, it is preferably carried out while heating the stretching rolls 63, 64 to heat the nonwoven fabric 51 to be processed. The heating temperature during this time is preferably higher than the preheating temperature during the preheating step, and a lower temperature than the melting point of the nonwoven fabric 51 to be processed.

In the shaping step, the upper stretching roll 63 forces its ridges 63a at the sections being in contact with the nonwoven fabric 51 to be processed into the hollow sections 64b of the lower stretching roll 64, thereby forming raised sections 4.

Meanwhile, the lower stretching roll 64 forces its plurality of pins 64a aligned in rows in the circumferential direction into the nonwoven fabric 51 to be processed being in contact with the tip sections of the pins 64a, at the corresponding grooves 63b of the upper stretching roll 63. The sections of the nonwoven fabric 51 to be processed that have been stretched in the grooves 63b, and have not been in contact with the pins 64a, become the furrow sections 5. Also, since the sections that have been in contact with the tip sections of the pins 64a are forcefully pushed into the grooves 63b and shaped, this forms the first perimeter wall sections 19 extending in the direction in which the raised sections 4 and furrow sections 5 are extended and the second perimeter wall sections 20 extending in the widthwise direction of the roll, as well as second hollow sections 16 with second bottom sections 18.

During formation, at the second bottom sections 18 of the second hollow sections 16, since the tip sections of the pins 64a push the contact sections of the nonwoven fabric 51 to be processed into the grooves 63b while the upper stretching roll 63 and lower stretching roll 64 mesh with the nonwoven fabric 51 to be processed, the fiber density is essentially higher than at the other sections and thus the rigidity is increased. Accordingly, due to the action of the second bottom sections 18 of the second hollow sections 16 in the nonwoven fabric 1, the rigidity of the nonwoven fabric 1 as a whole increases.

Furthermore, in the sections of the nonwoven fabric 51 to be processed that were in contact with both edges of the tip sections of the pins 64a in the widthwise direction (widthwise direction of the roll), with the help of the tension produced when the ridges 63a push the nonwoven fabric 51 to be processed in the direction of the lower stretching roll 64, the pins 64a shove aside the thermoplastic resin fibers forming the first perimeter wall sections 19 or break the fibers to form the broken fibers 23 with broken ends 23a.

This results in formation of hole sections 21 including the peripheral sections 22 that include the broken ends 23a of the broken fibers 23, in the second hollow sections 16. Incidentally, some of the thermoplastic resin fibers remain in a state passing through the interior spaces 21a of the hole sections 21 (passing thermoplastic resin fibers 24), and some of the broken ends 23a of the broken fibers 23 extend into the interior spaces 21a.

Since the hole sections 21 are formed in the sections of the perimeter wall sections 17 running along the machine direction MD of the nonwoven fabric 51 to be processed, i.e. the rotational direction of the stretching rolls 63, 64, which is the direction in which the raised sections 4 and the furrow sections 5 are extended, the hole sections 21 are formed in the first perimeter wall sections 19 running along the direction in which the raised sections 4 and furrow sections 5 are extended.

In the nonwoven fabric after the finish of the shaping step, the raised sections 4 and the furrow sections 5 are formed and therefore the nonwoven fabric for an absorbent article 1 is completed. The nonwoven fabric 1 is then subsequently used for a section that is in contact with skin, such as the top sheet or anti-leakage wall of an absorbent article such as a disposable diaper, sanitary napkin, incontinence pad, panty liner or the like, or it is attached to the outer surface of the back sheet of a disposable diaper.

In the nonwoven fabric for an absorbent article 1 having this configuration, since the mean fiber size of the fibers 4b forming the raised sections 4 is smaller than the distance between adjacent protrusions 31 of the fingerprint of the finger 30, the fibers 4b forming the raised sections 4 can infiltrate between the adjacent protrusions 31, 31 of fingerprints. Furthermore, by specifying the predetermined range for the distance between the top section 4a of the raised section 4 of the nonwoven fabric 1 and the top section 4a of raised section 4 adjacent to the raised section 4, and the average interfiber distance of the fibers 4b forming the raised sections 4, it is easier for the raised sections 4 as a whole to run along the finger 30 and the irregularities of the fingerprint of the finger 30, such that the fibers 4b forming the raised sections 4 can even more easily infiltrate into the adjacent protrusions 31, 31 of the fingerprint of the finger 30. Moreover, the presence of the second hollow sections 16 reduces the area of contact of the first bottom sections 12 of the first hollow sections 11 with the skin, so that a soft feel on the skin can be obtained.

This allows a soft feel on the skin to be obtained for the nonwoven fabric 1 as a whole, while also allowing the fibers 4b forming the raised sections 4 to stimulate the tactile organs on the flat sections 32 between adjacent protrusions 31, 31 of the fingerprint of the finger 30 and on the side sections 31a of the protrusions 31, to obtain a delicate, soft tactile sensation and a smooth tactile sensation.

In particular, in regard to the raised sections 4, the hole sections 21 of the first perimeter wall sections 19 of the perimeter wall sections 17 release tension of the fibers forming the adjacent raised sections 4, improving the freedom of movement of the raised sections 4 as a whole or the fibers 4b forming the raised sections 4, and therefore the flexibility of the raised sections 4 is increased. Consequently, when a finger 30 has touched the raised sections 4, the raised sections 4 flexibly deform by the external force and fit into the finger 30, while the fibers 4b forming the raised sections 4 easily follow the irregularities of the fingerprint of the finger 30 due to the high degree of freedom of the fibers 4b forming the raised sections 4. This allows a delicate, soft and smooth feel on the skin to be obtained in a more stable manner.

According to the above-mentioned embodiment, the first perimeter wall section 19 of the second hollow section 16 includes hole section 21 running through between the first surface 2 and the second surface 3, but it is not absolutely necessary to provide the holes.

In the above-mentioned embodiment, the hole sections 21 are provided in the first perimeter wall sections 19, but the configuration may be such that the hole sections are provided on the pair of second perimeter wall sections formed along the second direction, or provided on only one perimeter wall section.

Also in the above-mentioned embodiment, the hole sections 21 are disposed at the locations near the second bottom sections 18 on the first perimeter wall sections 19, but the locations of the hole sections on the perimeter wall sections do not need to be near the bottom sections, and may be set as desired within a range that does not impair the flexibility or rigidity of the nonwoven fabric.

Moreover in the above-mentioned embodiment, the thermoplastic resin fibers 24 are present passing through the interior spaces 21a of the hole sections 21, but thermoplastic resin fibers may not be present passing through the interior spaces of the hole sections in this manner. In addition, the broken ends of the broken fibers do not need to extend into the interior spaces of the hole sections as in the above-mentioned embodiment.

In the above-mentioned embodiment, the second hollow section 16 is formed as the approximately cuboid shape, but the shape of the second hollow section may be any desired shape such as cylindrical or square columnar.

Furthermore, the raised sections 4 in the above-mentioned embodiment are extended continuously in the first direction X of the nonwoven fabric 1. However, the raised sections do not need to be extended continuously in the first direction of the nonwoven fabric and may instead be intermittent. Nevertheless, the second hollow section is preferably provided at the first bottom section of the first hollow section, sandwiched between sections where adjacent raised sections are mutually continuous, as this will help the raised sections come into contact with the skin first while also impeding contact with the second hollow sections, thereby minimizing any sensation of foreign object or uncomfortable feeling due to the second hollow sections.

According to the above-mentioned embodiment, the fibers 4b forming the raised sections 4 of the nonwoven fabric 1 have the mean fiber size of 10 to 30 $\mu$m and the average interfiber distance of 50 to 150 $\mu$m, but the fibers forming the other sections, such as the furrow sections of the nonwoven fabric, may also have a mean fiber size or average interfiber distance in the same range as the fibers forming the raised sections.

EXAMPLES

In order to confirm the performance of the nonwoven fabric for an absorbent article of the present invention, a comparative experiment was conducted with a nonwoven fabric according to the present invention (example) and a nonwoven fabric not having the configuration of the present invention (comparative example).

As a comparative experiment, the examples and comparative examples were evaluated by a tester touching the top surface of the nonwoven fabric sample with a finger (the side on which the raised sections protruded), as to whether stroking the sample in the lengthwise direction and widthwise direction produced a tactile sensation of "moist", "smooth", "dry smooth" or "other", and the results were compared.

In organoleptic evaluation in the comparative experiment, the feel on the skin was categorized into "moist": i.e.,

skin-friendly and not actually wet but a feel on the skin reminiscent of light moistness, "smooth": i.e., a feel on the skin having a smooth touch and low roughness, and "dry smooth": i.e., a dry feel on the skin without any moist feeling. Here, "moist" feel on the skin and "dry smooth" feel on the skin are opposite tactile sensations, while "smooth" is a tactile sensation intermediate between "moist" and "dry smooth".

In the comparative experiment, two different samples including nonwoven fabrics of the present invention (hereunder referred to as Example 1 and Example 2), and three different samples that were not nonwoven fabrics according to the present invention (hereunder referred to as Comparative Example 1, Comparative Example 2 and Comparative Example 3) were prepared. As the samples, the nonwoven fabrics to be examined, cut to the size of 100 mm × 100 mm were used.

Also, the nonwoven fabric of the present invention had basically the same configuration as the above-mentioned embodiment, and it was produced by the same production method. Example 1 and Example 2 were different from each other in terms of the distance between the top section of the raised section and the top section of other raised section adjacent to the raised section (or pitch, hereunder referred to as "distance between raised sections"), and the mean fiber size of the fibers forming the raised sections.

On the other hand, the nonwoven fabric of Comparative Example 1 corresponded to the nonwoven fabric to be processed used for production of a nonwoven fabric of the present invention in the above-mentioned embodiment, being not shaped and being in an essentially flat form overall. The nonwoven fabric of Comparative Example 1 had the same overall mean fiber size as the mean fiber size of the fibers forming the raised sections in Example 1.

Also, the nonwoven fabric of Comparative Example 2 was shaped by a pair of mutually interlocking shaping rolls with ridges and grooves according to the above-mentioned embodiment and its cross-section had approximately wavy irregularities. The method of producing the nonwoven fabric of Comparative Example 2 was basically the same as for Example 1 and Example 2, in regard to the elements other than shaping. Moreover, the nonwoven fabric of Comparative Example 2 had the same mean fiber size for the fibers forming the raised sections as Example 1, but the structure of the nonwoven fabric and the distance between raised sections was different from Example 1.

Furthermore, the nonwoven fabric of Comparative Example 3 had basically the same structure as explained for the above-mentioned embodiment, and was produced by the same method, but the mean fiber size of the fibers forming the raised sections was not within the range of the invention, and the fibers composing the nonwoven fabric were also different from Example 1 and Example 2. The distance between raised sections in Comparative Example 3 was the same as Example 1.

For Example 1, Example 2, Comparative Example 1 and Comparative Example 2, the nonwoven fabric formed of PET/PE core-sheath composite fibers (basis weight: 30 g/m$^2$) was used, while for Comparative Example 3 the nonwoven fabric formed of PET/PP core-sheath composite fibers (basis weight: 30 g/m$^2$) was used. Examples 1 and 2 and Comparative Examples 1 to 3 were all through-air nonwoven fabrics, while for Examples 1 and 2 and Comparative Examples 2 and 3, in the preheating step and shaping step, a heating temperature is 100°C and the shaping was carried out with a transport speed of 250 m/min.

Also, the distance between raised sections and the mean fiber size of the fibers forming the raised sections were measured by the measurement methods described in the above-mentioned embodiment.

The results of the comparative experiment are shown in the following table. In the table, a value of 1 in the "Organoleptic evaluation" column stands for "moist", a value of 2 stands for "smooth" and a value of 3 stands for "dry smooth".

[Table 1]

|  | Example 1 | Example 2 | Comp. Example 1 | Comp. Example 2 | Comp. Example 3 |
|---|---|---|---|---|---|
| Distance between raised sections (pitch) (mm) | 1.5 | 2.0 | No irregularities | 3.0 | 1.5 |
| Fiber component | PE/PET | PE/PET | PE/PET | PE/PET | PE/PP |
| Mean fiber size of fibers forming raised sections (μm) | 12.8 | 18.7 | 12.8 | 12.8 | 32 |

(continued)

|  |  | Example 1 | Example 2 | Comp. Example 1 | Comp. Example 2 | Comp. Example 3 |
|---|---|---|---|---|---|---|
| Organoleptic evaluation | n1 | 1 | 1 | 2 | 3 | 3 |
|  | n2 | 1 | 2 | 3 | 3 | 3 |
|  | n3 | 1 | 1 | 3 | 3 | 3 |
|  | n4 | 1 | 2 | 2 | 3 | 3 |
|  | n5 | 1 | 1 | 3 | 2 | 3 |

As seen from Table 1, Example 1 produced a feel on the skin evaluated as "moist" by all of the testers, while Example 2 also had many testers noting a feel on the skin of "moist", with no testers indicating a feel on the skin of "dry smooth". However, in comparing Example 1 and Example 2, while Example 1 had more testers indicating a feel on the skin of "moist", this is presumably because the distance between raised sections in Example 2 was the upper limit of the range of the invention, and therefore Example 1, wherein the distance between raised sections was further from the upper limit or lower limit of the range of the present invention, more readily produced a feel on the skin of "moist".

On the other hand, for Comparative Examples 1 to 3, none of the testers indicated a feel on the skin of "moist". For Comparative Example 1, the mean fiber size was within the range of the present invention, but presumably because it did not have the basic structure of the nonwoven fabric of the present invention (for this example, this means the elements relating to the basic shape of the nonwoven fabric (elements other than the distance between raised sections and the mean fiber size of the fibers forming the raised sections), i.e. the structure of a nonwoven fabric having raised sections and first hollow sections and second hollow sections in furrow sections), a feel on the skin of "moist" was not obtained.

Comparative Example 2 did not have the basic structure of a nonwoven fabric of the present invention, and also had a distance between raised sections outside of the range of the present invention, and therefore even though it was a nonwoven fabric having a concavoconvex structure and the mean fiber size of the fibers forming the raised sections was within the range of the invention, presumably the lack of the basic structure of a nonwoven fabric according to the present invention and the distance between raised sections that was outside of the range of the invention made it impossible to obtain a feel on the skin of "moist".

Comparative Example 3 had the same basic structure as the present invention, but presumably because the mean fiber size of the fibers forming the raised sections was outside of the range of the present invention, it was not possible to obtain a feel on the skin of "moist".

As explained above, it has been demonstrated that the nonwoven fabric for an absorbent article according to the present invention can produce a feel on the skin of "moist".

REFERENCE SIGNS LIST

1 Nonwoven fabric for absorbent article
2 First surface of nonwoven fabric
3 Second surface of nonwoven fabric
4 Raised section
4a Top section of raised section
5 Furrow section
11 First hollow section
12 First bottom section
16 Second hollow section
17 Perimeter wall section
18 Second bottom section
19 First perimeter wall section
20 Second perimeter wall section
21 Hole section

**Claims**

1. A nonwoven fabric (1) for an absorbent article, containing thermoplastic resin fibers, having a first surface (2) and a second surface (3) opposing the first surface, and comprising: a plurality of raised sections (4) protruding in a direction from the second surface to the first surface; and a plurality of furrow sections (5) depressed in the direction from the first surface to the second surface,

   wherein the raised sections are extended in a first direction (X) of planar directions of the nonwoven fabric (1), while being provided at predetermined intervals in a second direction (Y) that is perpendicular to the first direction (X) of the planar directions of the nonwoven fabric,
   the furrow sections are extended in the first direction (X), in the spaces between mutually adjacent raised sections (4, 4) in the second direction (Y), and each includes a first hollow (11) section comprising a first bottom section (12) located further in the direction of the second surface (3) than the location of the first surface (2) at the top section (4a) of the raised section (4), and a plurality of second hollow sections (16) provided in a discontinuous manner in the first direction (X) in the first hollow section (11), formed as depressions opening into the first bottom section (12) and comprising second bottom sections (18) located further in the direction of the second surface (3) than the first bottom section (12),
   a distance between a top section (4a) of a raised section (4) and a top section (4a) of another raised section (4) adjacent to the raised section is 0.5 to 2 mm, and
   a mean fiber size of fibers (4b) forming at least the raised sections (4) of the nonwoven fabric is 10 to 30 $\mu$m.

2. The nonwoven fabric for an absorbent article according to claim 1, wherein an average interfiber distance of the fibers (4b) forming the raised sections (4) is 50 to 150 $\mu$m.

3. The nonwoven fabric for an absorbent article according to claim 1 or 2, wherein the second hollow section (16) has a perimeter wall section (17) extended in a direction opposite to the direction in which the raised section (4) protrude from the first bottom section (12), and the second bottom section (18) disposed on an edge of the perimeter wall section (17) in the direction opposite to the first bottom section (12) so as to plug the edge.

4. The nonwoven fabric for an absorbent article according to claim 3, wherein the perimeter wall section (17) of the second hollow section (16) has a pair of first perimeter wall sections (19) formed along the first direction (X), and a pair of second perimeter wall sections (20) formed along the second direction (Y), the first perimeter wall section (19) has a hole section (21) running through between the first surface (2) and the second surface (3).

5. The nonwoven fabric for an absorbent article according to claim 4, wherein the hole section (21) has a peripheral section (22) formed without melting of the thermoplastic resin fibers, and the peripheral section includes broken ends (23a) of broken fibers (23) that have broken ends formed by breakage among the thermoplastic resin fibers.

6. The nonwoven fabric for an absorbent article according to claim 4 or 5, wherein the hole section (21) has some of the thermoplastic resin fibers (24) passing through the interior space (21a) of the hole section.

7. The nonwoven fabric for an absorbent article according to any one of claims 4 to 6, wherein the hole section (21) has an open area ratio in the interior space (21a) of 1 to 50%.

8. The nonwoven fabric for an absorbent article according to any one of claims 4 to 7 wherein the hole sections (21) are provided only at locations near the second bottom sections (18) of the first perimeter wall sections (19).

9. The nonwoven fabric for an absorbent article according to any one of claims 1 to 8, wherein the second hollow section (16) has a distance of 0.05 to 2 mm from the first surface (2) of the first bottom section (12) of the first hollow section (11) to the first surface (2) of the second bottom section (18).

**Patentansprüche**

1. Vliesstoff (1) für einen absorbierenden Artikel, der thermoplastische Harzfasern enthält, eine erste Oberfläche (2) und eine zweite Oberfläche (3), die der ersten Obenfläche gegenüberliegt, aufweist und Folgendes umfasst: eine Vielzahl von erhöhten Abschnitten (4), die in einer Richtung von der zweiten Oberfläche zu der ersten Oberfläche hervorstehen; und eine Vielzahl von Rillenabschnitten (5), die in der Richtung von der ersten Oberfläche zu der zweiten Oberfläche eingedrückt sind,

wobei sich die erhöhten Abschnitte in einer ersten Richtung (X) von planaren Richtungen des Vliesstoffs (1) erstrecken, während sie in vorgegebenen Abständen in einer zweiten Richtung (Y) bereitgestellt sind, die senkrecht zu der ersten Richtung (X) der planaren Richtungen des Vliesstoffs verläuft, die Rillenabschnitte sich in der ersten Richtung (X) in dem Raum zwischen beidseitig benachbarten erhöhten Abschnitten (4, 4) in der zweiten Richtung (Y) erstrecken und jeder Folgendes einschließt: einen ersten ausgesparten (11) Abschnitt, der einen ersten unteren Abschnitt (12) umfasst, der sich weiter in der Richtung der zweiten Oberfläche (3) als die Stelle der ersten Oberfläche (2) an dem oberen Abschnitt (4a) des erhöhten Abschnitts (4) befindet, und eine Vielzahl von zweiten ausgesparten Abschnitten (16), die diskontinuierlich in der ersten Richtung (X) in dem ersten ausgesparten Abschnitt (11) bereitgestellt sind, als Vertiefungen ausgebildet sind, die sich in den ersten unteren Abschnitt (12) öffnen, und zweite untere Abschnitte (18) umfassen, die sich weiter in der Richtung der zweiten Oberfläche (3) als der erste untere Abschnitt (12) befinden, ein Abstand zwischen einem oberen Abschnitt (4a) eines erhöhten Abschnitts (4) und einem oberen Abschnitt (4a) eines anderen erhöhten Abschnitts (4), der benachbart zu dem erhöhten Abschnitt vorliegt, 0,5 bis 2 mm beträgt und eine mittlere Fasergröße von Fasern (4b), die zumindest die erhöhten Abschnitte (4) des Vliesstoffs ausbilden, 10 bis 30 $\mu$m beträgt.

2. Vliesstoff für einen absorbierenden Artikel nach Anspruch 1, wobei ein durchschnittlicher Zwischenfaserabstand der Fasern (4b), die die erhöhten Abschnitte (4) ausbilden, 50 bis 150 $\mu$m beträgt.

3. Vliesstoff für einen absorbierenden Artikel nach Anspruch 1 oder 2, wobei der zweite ausgesparte Abschnitt (16) Folgendes aufweist: einen Umfangswandabschnitt (17), der sich in einer Richtung gegenüberliegend zu der Richtung erstreckt, in der der erhöhte Abschnitt (4) von dem ersten unteren Abschnitt (12) hervorsteht, und den zweiten unteren Abschnitt (18), der an einem Rand des Umfangswandabschnitts (17) in der Richtung gegenüberliegend zu dem ersten unteren Abschnitt (12) derart angeordnet ist, dass der Rand ausgefüllt ist.

4. Vliesstoff für einen absorbierenden Artikel nach Anspruch 3, wobei der Umfangswandabschnitt (17) des zweiten ausgesparten Abschnitts (16) Folgendes aufweist: ein Paar von ersten Umfangswandabschnitten (19), die entlang der ersten Richtung (X) ausgebildet sind, und ein Paar von zweiten Umfangswandabschnitten (20), die entlang der zweiten Richtung (Y) ausgebildet sind, wobei der erste Umfangswandabschnitt (19) einen Lochabschnitt (21) aufweist, der zwischen der ersten Oberfläche (2) und der zweiten Oberfläche (3) durchgeht.

5. Vliesstoff für einen absorbierenden Artikel nach Anspruch 4, wobei der Lochabschnitt (21) einen umlaufenden Abschnitt (22) aufweist, der ohne Schmelzen der thermoplastischen Harzfasern ausgebildet ist, und der umlaufende Abschnitt abgebrochene Enden (23a) von gebrochenen Fasern (23) einschließt, die abgebrochene Enden aufweisen, die durch Brechen unter den thermoplastischen Harzfasern ausgebildet werden.

6. Vliesstoff für einen absorbierenden Artikel nach Anspruch 4 oder 5, wobei im Lochabschnitt (21) einige der thermoplastischen Harzfasern (24) durch den inneren Raum (21a) des Lochabschnitts durchlaufen.

7. Vliesstoff für einen absorbierenden Artikel nach einem der Ansprüche 4 bis 6, wobei der Lochabschnitt (21) einen Anteil an offener Fläche in dem inneren Raum (21a) von 1 bis 50 % aufweist.

8. Vliesstoff für einen absorbierenden Artikel nach einem der Ansprüche 4 bis 7, wobei die Lochabschnitte (21) nur an Stellen nahe der zweiten unteren Abschnitte (18) der ersten Umfangswandabschnitte (19) bereitgestellt sind.

9. Vliesstoff für einen absorbierenden Artikel nach einem der Ansprüche 1 bis 8, wobei der zweite ausgesparte Abschnitt (16) einen Abstand von 0,05 bis 2 mm von der ersten Oberfläche (2) des ersten unteren Abschnitts (12) des ersten ausgesparten Abschnitts (11) zu der ersten Oberfläche (2) des zweiten unteren Abschnitts (18) aufweist.

## Revendications

1. Tissu non tissé (1) pour un article absorbant, contenant des fibres de résine thermoplastique, ayant une première surface (2) et une deuxième surface (3) opposée par rapport à la première surface, et comportant : une pluralité de sections en relief (4) faisant saillie dans une direction allant de la deuxième surface jusqu'à la première surface ; et une pluralité de sections à sillons (5) déprimées dans la direction allant de la première surface jusqu'à la deuxième surface,

dans lequel les sections en relief sont étendues dans une première direction (X) de directions planes du tissu non tissé (1), tout en étant mises en œuvre selon des intervalles prédéterminés dans une deuxième direction (Y) qui est perpendiculaire par rapport à la première direction (X) des directions planes du tissu non tissé, les sections à sillons sont étendues dans la première direction (X), dans les espaces entre des sections en relief mutuellement adjacentes (4, 4) dans la deuxième direction (Y), et chacune comprend une première section creuse (11) comportant une première section inférieure (12) située davantage dans la direction de la deuxième surface (3) par rapport à l'emplacement de la première surface (2) au niveau de la section supérieure (4a) de la section en relief (4), et une pluralité de deuxièmes sections creuses (16) mises en œuvre de manière discontinue dans la première direction (X) dans la première section creuse (11), formées sous la forme de dépressions donnant dans la première section inférieure (12) et comportant des deuxièmes sections inférieures (18) situées davantage dans la direction de la deuxième surface (3) par rapport à la première section inférieure (12), une distance entre une section supérieure (4a) d'une section en relief (4) et une section supérieure (4a) d'une autre section en relief (4) adjacente par rapport à la section en relief va de 0,5 à 2 mm, et une grosseur moyenne de fibre des fibres (4b) formant au moins les sections en relief (4) du tissu non tissé va de 10 à 30 $\mu$m.

2. Tissu non tissé pour un article absorbant selon la revendication 1, dans lequel une distance moyenne entre fibres des fibres (4b) formant les sections en relief (4) va de 50 à 150 $\mu$m.

3. Tissu non tissé pour un article absorbant selon la revendication 1 ou la revendication 2, dans lequel la deuxième section creuse (16) a une section de paroi périmétrique (17) étendue dans une direction opposée par rapport à la direction dans laquelle la section en relief (4) fait saillie depuis la première section inférieure (12), et la deuxième section inférieure (18) disposée sur un bord de la section de paroi périmétrique (17) dans la direction opposée par rapport à la première section inférieure (12) de manière à boucher le bord.

4. Tissu non tissé pour un article absorbant selon la revendication 3, dans lequel la section de paroi périmétrique (17) de la deuxième section creuse (16) a une paire de premières sections de paroi périmétrique (19) formées le long de la première direction (X), et une paire de deuxièmes sections de paroi périmétrique (20) formées le long de la deuxième direction (Y), la première section de paroi périmétrique (19) a une section à trou (21) s'étendant entre la première surface (2) et la deuxième surface (3).

5. Tissu non tissé pour un article absorbant selon la revendication 4, dans lequel la section à trou (21) a une section périphérique (22) formée sans fusion des fibres de résine thermoplastique, et la section périphérique comprend des extrémités cassées (23a) de fibres cassées (23) qui ont des extrémités cassées formées par de la rupture parmi les fibres de résine thermoplastique.

6. Tissu non tissé pour un article absorbant selon la revendication 4 ou la revendication 5, dans lequel la section à trou (21) a certaines des fibres de résine thermoplastique (24) qui passent au travers de l'espace intérieur (21a) de la section à trou.

7. Tissu non tissé pour un article absorbant selon l'une quelconque des revendications 4 à 6, dans lequel la section à trou (21) a une proportion de zone ouverte dans l'espace intérieur (21a) de 1 à 50 %.

8. Tissu non tissé pour un article absorbant selon l'une quelconque des revendications 4 à 7, dans lequel les sections à trou (21) sont mises en œuvre uniquement au niveau d'emplacements à proximité des deuxièmes sections inférieures (18) des premières sections de paroi périmétrique (19).

9. Tissu non tissé pour un article absorbant selon l'une quelconque des revendications 1 à 8, dans lequel la deuxième section creuse (16) a une distance allant de 0,05 à 2 mm depuis la première surface (2) de la première section inférieure (12) de la première section creuse (11) jusqu'à la première surface (2) de la deuxième section inférieure (18).

# FIG. 1

# FIG. 2

FIG. 3

# FIG. 4

## (a)

## (b)

# FIG. 5

FIG. 6

# FIG. 7

# FIG. 8

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2005334374 A
- EP 2612961 A1
- WO 2013005782 A1